# EUROPEAN PATENT SPECIFICATION

(11) **EP 2 401 341 B1**
(45) Date of publication and mention of the grant of the patent: **18.01.2017**
(21) Application number: 10746974.4
(22) Date of filing: 01.03.2010
(51) Int. Cl.: H01L 51/54, C09K 11/06, C07C 15/28, C07C 211/54

(54) **DEUTERATED COMPOUNDS FOR ELECTRONIC APPLICATIONS**
DEUTERIERTE VERBINDUNGEN FÜR ELEKTRONISCHE ANWENDUNGEN
COMPOSÉS DEUTÉRÉS POUR APPLICATIONS ÉLECTRONIQUES

(30) Priority: 27.02.2009 US 156181 P
(43) Date of publication of application: 04.01.2012
(73) Proprietor: E. I. du Pont de Nemours and Company, Wilmington, DE 19805 (US)
(72) Inventor: LECLOUX, Daniel, David, West Chester ,Pennsylvania 19382 (US); FENNIMORE, Adam, Wilmington Delaware 19808 (US); GAO, Weiying, Landenberg Pennsylvania 19350 (US); RADU, Nora, Sabina, Landenberg Pennsylvania 19350 (US); WU, Weishi, Landenberg Pennsylvania 19350 (US); ROSTOVTSEV, Vsevolod, Swarthmore Pennsylvania 19081 (US); HOWARD, Michael, Henry, Jr., Montchanin Delaware 19710 (US)
(74) Representative: Towler, Philip Dean
(86) International application number: PCT/US2010/025764
(87) International publication number: WO 2010/099534

(56) References cited:
- WO-A1-2007/105917
- WO-A1-2007/108666
- WO-A1-2007/108666
- KR-A- 20090 086 015
- US-A1- 2006 115 678
- US-A1- 2006 115 678
- US-A1- 2008 191 614
- US-B2- 7 173 131

## Description

### RELATED APPLICATION

This application claims priority under 35 U.S.C. § 119(e) from U.S. Provisional Application No. 61/156, 181 filed on February 27, 2009.

### BACKGROUND

### Field of the Disclosure

This disclosure relates to anthracene derivative compounds which are at least partially deuterated. It also relates to electronic devices in which the active layers include such a compound.

### Description of the Related Art

Organic electronic devices that emit light, such as light-emitting diodes that make up displays, are present in many different kinds of electronic equipment. In all such devices, an organic active layer is sandwiched between two electrical contact layers. At least one of the electrical contact layers is light-transmitting so that light can pass through the electrical contact layer. The organic active layer emits light through the light-transmitting electrical contact layer upon application of electricity across the electrical contact layers.

It is well known to use organic electroluminescent compounds as the active component in light-emitting diodes. WO 2007/105917 A1 and WO 2007/108666 A1 disclose aryl-substituted anthracene derivatives having deuterated substituents. US 2006/0115678 A1 discloses deuterated aminoathryl compounds. Simple organic molecules such as anthracene, thiadiazole derivatives, and coumarin derivatives are known to show electroluminescence. Semiconductive conjugated polymers have also been used as electroluminescent components, as has been disclosed in, for example, U.S. Patent 5,247,190, U.S. Patent 5,408,109, and Published European Patent Application 443 861.

In many cases the electroluminescent compound is present in a host material. There is a continuing need for new host compounds.

### SUMMARY

There is provided an aryl-substituted anthracene having at least one D.

There is also provided an electronic device comprising an active layer comprising the above compound.

### BRIEF DESCRIPTION OF THE DRAWINGS

Embodiments are illustrated in the accompanying figures to improve understanding of concepts as presented herein.
FIG. 1 includes an illustration of one example of an organic electronic device.

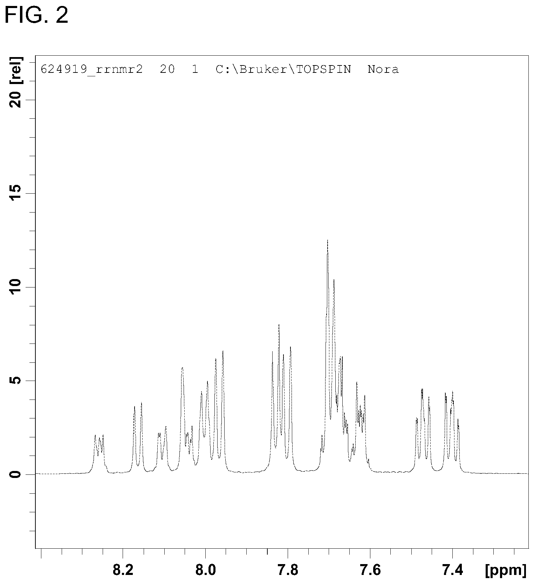

FIG. 2 includes the ¹H NMR spectrum of the comparative compound of Comparative Example A.
FIG. 3 includes the ¹H NMR spectrum of the deuterated compound of Example 1.
FIG. 4 includes the mass spectrum of the deuterated compound of Example 1.

Skilled artisans appreciate that objects in the figures are illustrated for simplicity and clarity and have not necessarily been drawn to scale. For example, the dimensions of some of the objects in the figures may be exaggerated relative to other objects to help to improve understanding of embodiments.

### DETAILED DESCRIPTION

Many aspects and embodiments are disclosed herein and are exemplary and not limiting. After reading this specification, skilled artisans appreciate that other aspects and embodiments are possible without departing from the scope of the invention.

Other features and benefits of any one or more of the embodiments will be apparent from the following detailed description, and from the claims. The detailed description first addresses Definitions and Clarification of Terms followed by the Deuterated Compound, the Electronic Device, and finally Examples.

### 1. Definitions and Clarification of Terms

Before addressing details of embodiments described below, some terms are defined or clarified.

As used herein, the term "aliphatic ring" is intended to mean a cyclic group that does not have delocalized pi electrons. In some embodiments, the aliphatic ring has no unsaturation. In some embodiments, the ring has one double or triple bond.

The term "alkoxy" refers to the group RO-, where R is an alkyl.

The term "alkyl" is intended to mean a group derived from an aliphatic hydrocarbon having one point of attachment, and includes a linear, a branched, or a cyclic group. The term is intended to include heteroalkyls. The term "hydrocarbon alkyl" refers to an alkyl group having no heteroatoms. The term "deuterated alkyl" is a hydrocarbon alkyl having at least one available H replaced by D. In some embodiments, an alkyl group has from 1-20 carbon atoms.

The term "branched alkyl" refers to an alkyl group having at least one secondary or tertiary carbon. The term "secondary alkyl" refers to a branched alkyl group having a secondary carbon atom. The term "tertiary alkyl" refers to a branched alkyl group having a tertiary carbon atom. In some embodiments, the branched alkyl group is attached via a secondary or tertiary carbon. Branched alkyl groups may also be deuterated.

The term "aryl" is intended to mean a group derived from an aromatic hydrocarbon having one point of attachment. The term "aromatic compound" is intended to mean an organic compound comprising at least one unsaturated cyclic group having delocalized pi electrons. The term is intended include heteroaryls. The term "hydrocarbon aryl" is intended to mean aromatic compounds having no heteroatoms in the ring. The term aryl includes groups which have a single ring and those which have multiple rings which can be joined by a single bond or fused together. The term "deuterated aryl" refers to an aryl group having at least one available H bonded directly to the aryl replaced by D. The term "arylene" is intended to mean a group derived from an aromatic hydrocarbon having two points of attachment. In some embodiments, an aryl group has from 3-60 carbon atoms. Arylene groups may be deuterated.

The term "aryloxy" refers to the group RO-, where R is an aryl.

The term "compound" is intended to mean an electrically uncharged substance made up of molecules that further consist of atoms, wherein the atoms cannot be separated by physical means. The phrase "adjacent to," when used to refer to layers in a device, does not necessarily mean that one layer is immediately next to another layer. On the other hand, the phrase "adjacent R groups," is used to refer to R groups that are next to each other in a chemical formula (i.e., R groups that are on atoms joined by a bond). The term "photoactive" refers to any material that exhibits electroluminescence and/or photosensitivity.

The term "deuterated" is intended to mean that at least one available H has been replaced by D.

The prefix "hetero" indicates that one or more carbon atoms have been replaced with a different atom. In some embodiments, the different atom is N, O, or S.

The term "layer" is used interchangeably with the term "film" and refers to a coating covering a desired area. The term is not limited by size. The area can be as large as an entire device or as small as a specific functional area such as the actual visual display, or as small as a single sub-pixel. Layers and films can be formed by any conventional deposition technique, including vapor deposition, liquid deposition (continuous and discontinuous techniques), and thermal transfer. Continuous deposition techniques, include but are not limited to, spin coating, gravure coating, curtain coating, dip coating, slot-die coating, spray coating, and continuous nozzle coating. Discontinuous deposition techniques include, but are not limited to, ink jet printing, gravure printing, and screen printing.

The term "organic electronic device" or sometimes just "electronic device" is intended to mean a device including one or more organic semiconductor layers or materials.

All groups can be substituted or unsubstituted unless otherwise indicated. In some embodiments, the substituents are selected from the group consisting of D, halide, alkyl, alkoxy, aryl, aryloxy, cyano, and NR₂, where R is alkyl or aryl.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

The IUPAC numbering system is used throughout, where the groups from the Periodic Table are numbered from left to right as 1-18 (CRC Handbook of Chemistry and Physics, 81st Edition, 2000).

### 2. Deuterated Compound

The new deuterated compound is an aryl-substituted anthracene compound having at least one D. In some embodiments, the compound is at least 10% deuterated. By this is meant that at least 10% of the available H are replaced by D. In some embodiments, the compound is at least 20% deuterated; in some embodiments, at least 30% deuterated; in some embodiments, at least 40% deuterated; in some embodiments, at least 50% deuterated; in some embodiments, at least 60% deuterated; in some embodiments, at least 70% deuterated; in some embodiments, at least 80% deuterated.

The deuterated compound has Formula I: 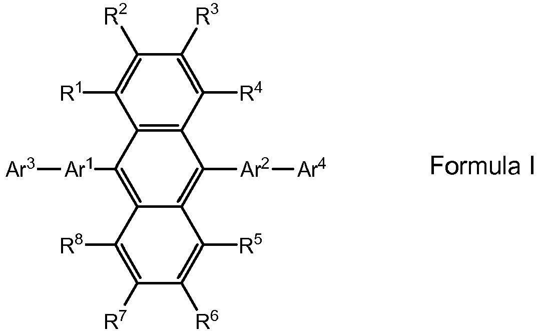 wherein:
R¹ through R⁸ are the same or different at each occurrence and are selected from the group consisting of H, D, alkyl, alkoxy, aryl, aryloxy, diarylamino, siloxane, and silyl;
Ar¹ and Ar² are the same or different and are selected from the group consisting of aryl groups; and
Ar³ and Ar⁴ are the same or different and are selected from the group consisting of H, D, and aryl groups;
provided that at least one of R¹ through R⁸ is D.

In some embodiments of Formula I, at least one D is on a substituent group on an aryl ring. In some embodiments, the substituent group is selected from alkyl, aryl, and diarylamino. In some embodiments, at least two of R¹ through R⁸ are D. In some embodiments, at least three are D; in some embodiments, at least four are D; in some embodiments, at least five are D; in some embodiments, at least six are D; in some embodiments, at least seven are D. In some embodiments, all of R¹ through R⁸ are D.

In some embodiments, R¹ through R⁸ are selected from H and D. In some embodiments, one of R¹ through R⁸ are D and seven are H. In some embodiments, two of R¹ through R⁸ are D and six are H. In some embodiments, three of R¹ through R⁸ are D and five are H. In some embodiments, four of R¹ through R⁸ are D, and four are H. In some embodiments, five of R¹ through R⁸ are D and three are H. In some embodiments, six of R¹ through R⁸ are D and two are H. In some embodiments, seven of R¹ through R⁸ are D and one is H.

In some embodiments, at least one of R¹ through R⁸ is selected from alkyl, alkoxy, aryl, aryloxy, diarylamino, siloxane, and silyl, and the remainder of R¹ through R⁸ are selected from H and D. In some embodiments, R² is selected from alkyl, alkoxy, aryl, aryloxy, diarylamino, siloxane, and silyl. In some embodiments, R² is selected from alkyl and aryl. In some embodiments, R² is selected from deuterated alkyl and deuterated aryl. In some embodiments, R² is selected from deuterated aryl having at least 50% deuteration. In some embodiments, R² is selected from deuterated aryl having at least 70% deuteration. In some embodiments, R² is selected from deuterated aryl having at least 80% deuteration.

In some embodiments of Formula I, at least one of Ar¹ through Ar⁴ is a deuterated aryl. In some embodiments, Ar³ and Ar⁴ are selected from D and deuterated aryls.

In some embodiments of Formula I, Ar¹ through Ar⁴ are at least 20% deuterated. In some embodiments of Formula I, Ar¹ through Ar⁴ are at least 40% deuterated; in some embodiments, at least 60% deuterated; in some embodiments, at least 80% deuterated.

In some embodiments, the compound of Formula I is at least 20% deuterated; in some embodiments, at least 30% deuterated; in some embodiments, at least 40% deuterated; in some embodiments, at least 50% deuterated; in some embodiments, at least 60% deuterated; in some embodiments, at least 70% deuterated; in some embodiments, at least 80% deuterated.

In some embodiments, Ar¹ and Ar² are selected from the group consisting of phenyl, naphthyl, phenanthryl, and anthracenyl. In some embodiments, Ar¹ and Ar² are selected from the group consisting of phenyl and naphthyl.

In some embodiments, Ar³ and Ar⁴ are selected from the group consisting of phenyl, naphthyl, phenanthryl, anthracenyl, phenylnaphthylene, naphthylphenylene, and a group having Formula II: 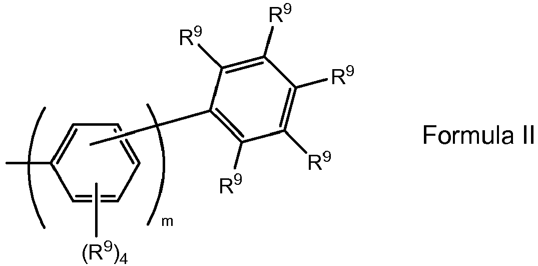 where:
R⁹ is the same or different at each occurrence and is selected from the group consisting of H, D, alkyl, alkoxy, diarylamino, siloxane and silyl, or adjacent R⁹ groups may be joined together to form an aromatic ring; and
m is the same or different at each occurrence and is an integer from 1 to 6.

In some embodiments, Ar³ and Ar⁴ are selected from the group consisting of phenyl, naphthyl, phenylnaphthylene, naphthylphenylene, and a group having Formula III: 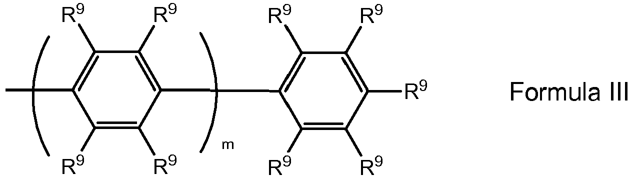 where R⁹ and m are as defined above for Formula II. In some embodiments, m is an integer from 1 to 3.

In some embodiments, at least one of Ar¹ through Ar⁴ is a heteroaryl group. In some embodiments, the heteroaryl group is selected from carbazole, benzofuran, and dibenzofuran. In some embodiments, the heteroaryl group is deuterated. In some embodiments, the heteroaryl group is at least 20% deuterated; in some embodiments, at least 40% deuterated; in some embodiments, at least 60% deuterated; in some embodiments, at least 80% deuterated.

In some embodiments of Formula I, at least one of R¹ through R⁸ is D and at least one of Ar¹ through Ar⁴ is a deuterated aryl. In some embodiments, the compound is at least 10% deuterated. In some embodiments, the compound is at least 20% deuterated; in some embodiments, at least 30% deuterated; in some embodiments, at least 40% deuterated; in some embodiments, at least 50% deuterated; in some embodiments, at least 60% deuterated; in some embodiments, at least 70% deuterated; in some embodiments, at least 80% deuterated.

Some non-limiting examples of compounds having Formula I include Compounds H1 through H7 below:
Compound H1: 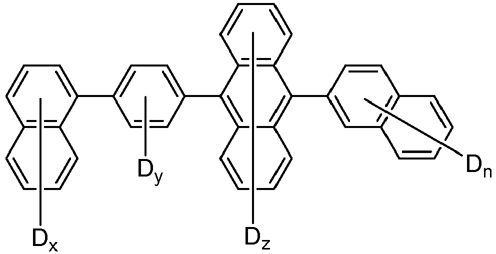 where x + y + z + n = 20-26
Compound H2: 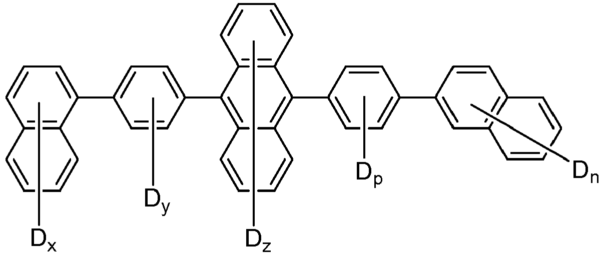 where x + y + z + p + n = 24-30
Compound H3: 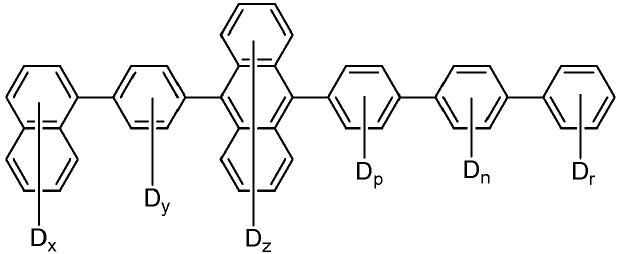 wherex+y+z+p+n+r=26-32
Compound H4: 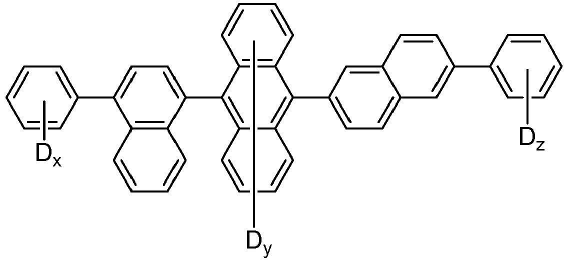 where x + y + z + p + n = 16-18
Compound H5: 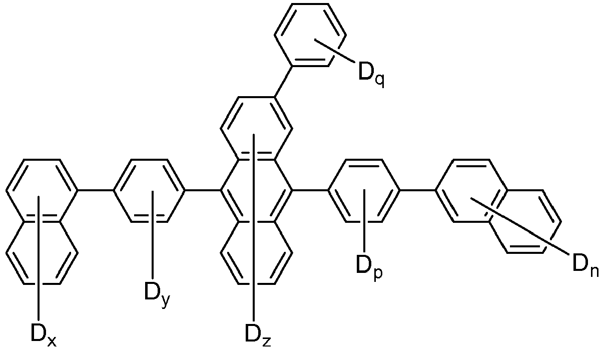 where x + y + z + p + n + q = 28-34
Compound H6 (not forming part of the invention): 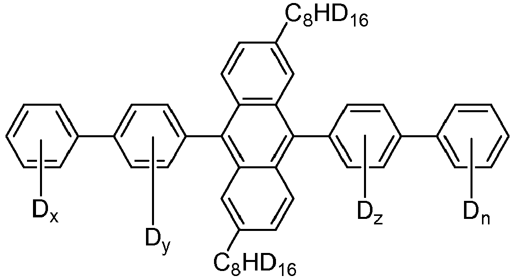 where x + y + z + n = 14-18
Compound H7: 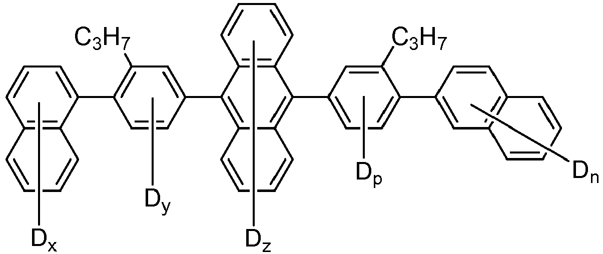 where x + y + z + p + n = 22-28

The non-deuterated analogs of the new compounds can be prepared by known coupling and substitution reactions. The new deuterated compound can then be prepared in a similar manner using deuterated precursor materials or, more generally, by treating the non-deuterated compound with deuterated solvent, such as d6-benzene, in the presence of a Lewis acid H/D exchange catalyst, such as aluminum trichloride or ethyl aluminum chloride. Exemplary preparations are given in the Examples. The level of deuteration can be determined by NMR analysis and by mass spectrometry, such as Atmospheric Solids Analysis Probe Mass Spectrometry (ASAP-MS).

The compounds described herein can be formed into films using liquid deposition techniques. Surprisingly and unexpectedly, these compounds have greatly improved properties when compared to analogous non-deuterated compounds. Electronic devices including an active layer with the compounds described herein, have greatly improved lifetimes. In addition, the lifetime increases are achieved in combination with high quantum efficiency and good color saturation. Furthermore, the deuterated compounds described herein have greater air tolerance than the non-deuterated analogs. This can result in greater processing tolerance both for the preparation and purification of the materials and in the formation of electronic devices using the materials.

### 3. Electronic Device

Organic electronic devices that may benefit from having one or more layers comprising the electroluminescent materials described herein include, but are not limited to, (1) devices that convert electrical energy into radiation (e.g., a light-emitting diode, light emitting diode display, or diode laser), (2) devices that detect signals through electronics processes (e.g., photodetectors, photoconductive cells, photoresistors, photoswitches, phototransistors, phototubes, IR detectors), (3) devices that convert radiation into electrical energy, (e.g., a photovoltaic device or solar cell), and (4) devices that include one or more electronic components that include one or more organic semi-conductor layers (e.g., a transistor or diode).

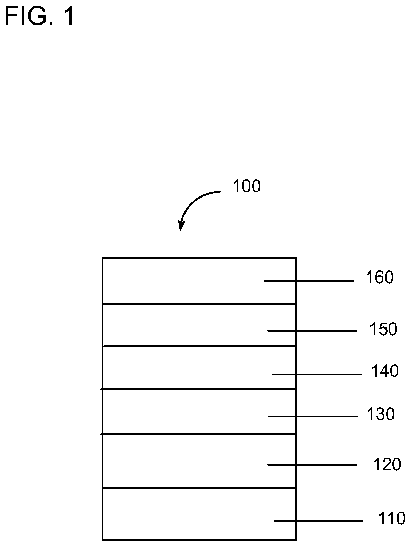

One illustration of an organic electronic device structure is shown in Figure 1. The device 100 has a first electrical contact layer, an anode layer 110 and a second electrical contact layer, a cathode layer 160, and a photoactive layer 140 between them. Adjacent to the anode is a buffer layer 120. Adjacent to the buffer layer is a hole transport layer 130, comprising hole transport material. Adjacent to the cathode may be an electron transport layer 150, comprising an electron transport material. As an option, devices may use one or more additional hole injection or hole transport layers (not shown) next to the anode 110 and/or one or more additional electron injection or electron transport layers (not shown) next to the cathode 160.

Layers 120 through 150 are individually and collectively referred to as the active layers.

In one embodiment, the different layers have the following range of thicknesses: anode 110, 50-500 nm, in one embodiment 100-200 nm; buffer layer 120, 5-200 nm, in one embodiment 20-100 nm; hole transport layer 130, 5-200 nm, in one embodiment 20-100 nm, photoactive layer 140, 1-200 nm, in one embodiment 10-100 nm; layer 150, 5-200 nm, in one embodiment 10-100 nm; cathode 160, 20-100 nm, in one embodiment 30-500nm. The location of the electron-hole recombination zone in the device, and thus the emission spectrum of the device, can be affected by the relative thickness of each layer. The desired ratio of layer thicknesses will depend on the exact nature of the materials used.

Depending upon the application of the device 100, the photoactive layer 140 can be a light-emitting layer that is activated by an applied voltage (such as in a light-emitting diode or light-emitting electrochemical cell), or a layer of material that responds to radiant energy and generates a signal with or without an applied bias voltage (such as in a photodetector). Examples of photodetectors include photoconductive cells, photoresistors, photoswitches, phototransistors, and phototubes, and photovoltaic cells, as these terms are described in Markus, John, Electronics and Nucleonics Dictionary, 470 and 476 (McGraw-Hill, Inc. 1966).

In some embodiments, the new deuterated compounds are useful as hole transport materials in layer 130. In some embodiments, at least one additional layer includes a deuterated material. In some embodiments, the additional layer is the buffer layer 120. In some embodiments, the additional layer is the photoactive layer 140. In some embodiments, the additional layer is the electron transport layer 150.

In some embodiments, the new deuterated compounds are useful as host materials for photoactive materials in photoactive layer 140. In some embodiments, the emissive material is also deuterated. In some embodiments, at least one additional layer includes a deuterated material. In some embodiments, the additional layer is the buffer layer 120. In some embodiments, the additional layer is the hole transport layer 130. In some embodiments, the additional layer is the electron transport layer 150

In some embodiments, the new deuterated compounds are useful as electron transport materials in layer 150. In some embodiments, at least one additional layer includes a deuterated material. In some embodiments, the additional layer is the buffer layer 120. In some embodiments, the additional layer is the hole transport layer 130. In some embodiments, the additional layer is the photoactive layer 140.

In some embodiments, an electronic device has deuterated materials in any combination of layers selected from the group consisting of the buffer layer, the hole transport layer, the photoactive layer, and the electron transport layer.

In some embodiments, the devices have additional layers to aid in processing or to improve functionality. Any or all of these layers can include deuterated materials. In some embodiments, all the organic device layers comprise deuterated materials. In some embodiments, all the organic device layers consist essentially of deuterated materials.

### a. Photoactive layer

The new deuterated compounds of Formula I are useful as hosts for photoactive materials in layer 140. The compounds can be used alone, or in combination with a second host material. The new deuterated compounds can be used as a host for materials with any color of emission. In some embodiments, the new deuterated compounds are used as hosts for green- or blue-emissive materials.

In some embodiments, the photoactive layer consists essentially of a host material having Formula I and one or more electroluminescent compounds.

Any EL material can be used in the devices, including, but not limited to, small molecule organic fluorescent compounds, fluorescent and phosphorescent metal complexes, conjugated polymers, and mixtures thereof. Examples of fluorescent compounds include, but are not limited to, chrysenes, pyrenes, perylenes, rubrenes, coumarins, anthracenes, thiadiazoles, derivatives thereof, and mixtures thereof. Examples of metal complexes include, but are not limited to, metal chelated oxinoid compounds, such as tris(8-hydroxyquinolato)aluminum (Alq3); cyclometalated iridium and platinum electroluminescent compounds, such as complexes of iridium with phenylpyridine, phenylquinoline, or phenylpyrimidine ligands as disclosed in Petrov et al., U.S. Patent 6,670,645 and Published PCT Applications WO 03/063555 and WO 2004/016710, and organometallic complexes described in, for example, Published PCT Applications WO 03/008424, WO 03/091688, and WO 03/040257, and mixtures thereof. Examples of conjugated polymers include, but are not limited to poly(phenylenevinylenes), polyfluorenes, poly(spirobifluorenes), polythiophenes, poly(p-phenylenes), copolymers thereof, and mixtures thereof.

In some embodiments, the photoactive dopant is a cyclometalated complex of iridium. In some embodiments, the complex has two ligands selected from phenylpyridines, phenylquinolines, and phenylisoquinolines, and a third liqand with is a β-dienolate. The ligands may be unsubstituted or substituted with F, D, alkyl, CN, or aryl groups.

In some embodiments, the photoactive dopant is a polymer selected from the group consisting of poly(phenylenevinylenes), polyfluorenes, and polyspirobifluorenes.

In some embodiments, the photoactive dopant is selected from the group consisting of a non-polymeric spirobifluorene compound and a fluoranthene compound.

In some embodiments, the photoactive dopant is a compound having aryl amine groups. In some embodiments, the photoactive dopant is selected from the formulae below: 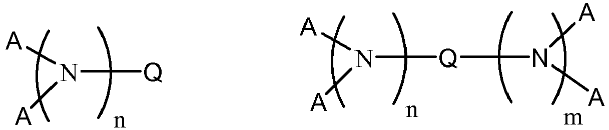 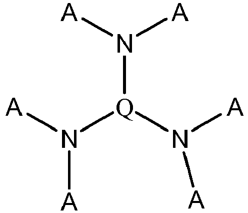 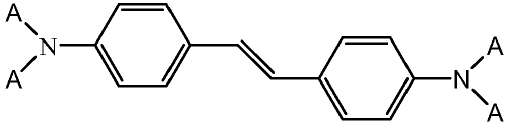 where:
A is the same or different at each occurrence and is an aromatic group having from 3-60 carbon atoms;
Q is a single bond or an aromatic group having from 3-60 carbon atoms;
n and m are independently an integer from 1-6.

In some embodiments of the above formula, at least one of A and Q in each formula has at least three condensed rings. In some embodiments, m and n are equal to 1.

In some embodiments, Q is a styryl or styrylphenyl group.

In some embodiments, Q is an aromatic group having at least two condensed rings. In some embodiments, Q is selected from the group consisting of naphthalene, anthracene, chrysene, pyrene, tetracene, xanthene, perylene, coumarin, rhodamine, quinacridone, and rubrene.

In some embodiments, A is selected from the group consisting of phenyl, tolyl, naphthyl, and anthracenyl groups.

In some embodiments, the photoactive dopant has the formula below: 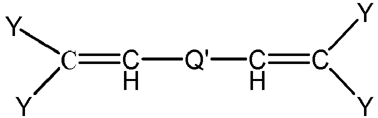 where:
Y is the same or different at each occurrence and is an aromatic group having 3-60 carbon atoms;
Q' is an aromatic group, a divalent triphenylamine residue group, or a single bond.

In some embodiments, the photoactive dopant is an aryl acene. In some embodiments, the photoactive dopant is a non-symmetrical aryl acene.

In some embodiments, the photoactive dopant is a chrysene derivative. The term "chrysene" is intended to mean 1,2-benzophenanthrene. In some embodiments, the photoactive dopant is a chrysene having aryl substituents. In some embodiments, the photoactive dopant is a chrysene having arylamino substituents. In some embodiments, the photoactive dopant is a chrysene having two different arylamino substituents. In some embodiments, the chrysene derivative has a deep blue emission.

In some embodiments, the photoactive dopant is selected from the group consisting of amino-substituted chrysenes and amino-substituted anthracenes.

### b. Other Device Layers

The other layers in the device can be made of any materials that are known to be useful in such layers.

The anode 110, is an electrode that is particularly efficient for injecting positive charge carriers. It can be made of, for example, materials containing a metal, mixed metal, alloy, metal oxide or mixed-metal oxide, or it can be a conducting polymer, or mixtures thereof. Suitable metals include the Group 11 metals, the metals in Groups 4-6, and the Group 8-10 transition metals. If the anode is to be light-transmitting, mixed-metal oxides of Groups 12, 13 and 14 metals, such as indium-tin-oxide, are generally used. The anode 110 can also comprise an organic material such as polyaniline as described in "Flexible light-emitting diodes made from soluble conducting polymer," Nature vol. 357, pp 477-479 (11 June 1992). At least one of the anode and cathode is desirably at least partially transparent to allow the generated light to be observed.

The buffer layer 120 comprises buffer material and may have one or more functions in an organic electronic device, including but not limited to, planarization of the underlying layer, charge transport and/or charge injection properties, scavenging of impurities such as oxygen or metal ions, and other aspects to facilitate or to improve the performance of the organic electronic device. Buffer materials may be polymers, oligomers, or small molecules. They may be vapour deposited or deposited from liquids which may be in the form of solutions, dispersions, suspensions, emulsions, colloidal mixtures, or other compositions.

The buffer layer can be formed with polymeric materials, such as polyaniline (PANI) or polyethylenedioxythiophene (PEDOT), which are often doped with protonic acids. The protonic acids can be, for example, poly(styrenesulfonic acid), poly(2-acrylamido-2-methyl-1-propanesulfonic acid), and the like.

The buffer layer can comprise charge transfer compounds, and the like, such as copper phthalocyanine and the tetrathiafulvalene-tetracyanoquinodimethane system (TTF-TCNQ).

In some embodiments, the buffer layer comprises at least one electrically conductive polymer and at least one fluorinated acid polymer. Such materials have been described in, for example, published U.S. patent applications 2004-0102577, 2004-0127637, and 2005/205860

In some embodiments, the hole transport layer 130 comprises the new deuterated compound of Formula I. Examples of other hole transport materials for layer 130 have been summarized for example, in Kirk-Othmer Encyclopedia of Chemical Technology, Fourth Edition, Vol. 18, p. 837-860, 1996, by Y. Wang. Both hole transporting molecules and polymers can be used. Commonly used hole transporting molecules are: N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis-(3-methylphenyl)-N,N,N',N'-2,5-phenylenediamine (PDA), a-phenyl-4-N,N-diphenylaminostyrene (TPS), p-(diethylamino)benzaldehyde diphenylhydrazone (DEH), triphenylamine (TPA), bis[4-(N,N-diethylamino)-2-methylphenyl](4-methylphenyl)methane (MPMP), 1-phenyl-3-[p-(diethylamino)styryl]-5-[p-(diethylamino)phenyl] pyrazoline (PPR or DEASP), 1,2-trans-bis(9H-carbazol-9-yl)cyclobutane (DCZB), N,N,N',N'-tetrakis(4-methylphenyl)-(1,1'-biphenyl)-4,4'-diamine (TTB), N,N'-bis(naphthalen-1-yl)-N,N'-bis-(phenyl)benzidine (-NPB), and porphyrinic compounds, such as copper phthalocyanine. Commonly used hole transporting polymers are polyvinylcarbazole, (phenylmethyl)-polysilane, and polyaniline. It is also possible to obtain hole transporting polymers by doping hole transporting molecules such as those mentioned above into polymers such as polystyrene and polycarbonate. In some cases, triarylamine polymers are used, especially triarylamine-fluorene copolymers. In some cases, the polymers and copolymers are crosslinkable. Examples of crosslinkable hole transport polymers can be found in, for example, published US patent application 2005-0184287 and published PCT application WO 2005/052027. In some embodiments, the hole transport layer is doped with a p-dopant, such as tetrafluorotetracyanoquinodimethane and perylene-3,4,9,10-tetracarboxylic-3,4,9,10-dianhydride.

In some embodiments, the electron transport layer 150 comprises the new deuterated compound of Formula I. Examples of other electron transport materials which can be used in layer 150 include metal chelated oxinoid compounds, such as tris(8-hydroxyquinolato)aluminum (Alq₃); bis(2-methyl-8-quinolinolato)(para-phenyl-phenolato)aluminum(III) (BAIQ); and azole compounds such as 2-(4-biphenylyl)-5-(4-t-butylphenyl)-1,3,4-oxadiazole (PBD) and 3-(4-biphenylyl)-4-phenyl-5-(4-t-butylphenyl)-1,2,4-triazole (TAZ), and 1,3,5-tri(phenyl-2-benzimidazole)benzene (TPBI); quinoxaline derivatives such as 2,3-bis(4-fluorophenyl)quinoxaline; phenanthroline derivatives such as 9,10-diphenylphenanthroline (DPA) and 2,9-dimethyl-4,7-diphenyl-1,10-phenanthroline (DDPA); and mixtures thereof. The electron-transport layer may also be doped with n-dopants, such as Cs or other alkali metals. Layer 150 can function both to facilitate electron transport, and also serve as a buffer layer or confinement layer to prevent quenching of the exciton at layer interfaces. Preferably, this layer promotes electron mobility and reduces exciton quenching.

The cathode 160, is an electrode that is particularly efficient for injecting electrons or negative charge carriers. The cathode can be any metal or nonmetal having a lower work function than the anode. Materials for the cathode can be selected from alkali metals of Group 1 (e.g., Li, Cs), the Group 2 (alkaline earth) metals, the Group 12 metals, including the rare earth elements and lanthanides, and the actinides. Materials such as aluminum, indium, calcium, barium, samarium and magnesium, as well as combinations, can be used. Li- or Cs-containing organometallic compounds, LiF, CsF, and Li₂O can also be deposited between the organic layer and the cathode layer to lower the operating voltage.

It is known to have other layers in organic electronic devices. For example, there can be a layer (not shown) between the anode 110 and buffer layer 120 to control the amount of positive charge injected and/or to provide band-gap matching of the layers, or to function as a protective layer. Layers that are known in the art can be used, such as copper phthalocyanine, silicon oxy-nitride, fluorocarbons, silanes, or an ultra-thin layer of a metal, such as Pt. Alternatively, some or all of anode layer 110, active layers 120, 130, 140, and 150, or cathode layer 160, can be surface-treated to increase charge carrier transport efficiency. The choice of materials for each of the component layers is preferably determined by balancing the positive and negative charges in the emitter layer to provide a device with high electroluminescence efficiency.

It is understood that each functional layer can be made up of more than one layer.

The device can be prepared by a variety of techniques, including sequential vapor deposition of the individual layers on a suitable substrate. Substrates such as glass, plastics, and metals can be used. Conventional vapor deposition techniques can be used, such as thermal evaporation, chemical vapor deposition, and the like. Alternatively, the organic layers can be applied from solutions or dispersions in suitable solvents, using conventional coating or printing techniques, including but not limited to spin-coating, dip-coating, roll-to-roll techniques, ink-jet printing, screenprinting, gravure printing and the like.

The present invention also relates to an electronic device comprising at least one active layer positioned between two electrical contact layers, wherein the at least one active layer of the device includes the anthracene compound of Formula 1. Devices frequently have additional hole transport and electron transport layers.

To achieve a high efficiency LED, the HOMO (highest occupied molecular orbital) of the hole transport material desirably aligns with the work function of the anode, and the LUMO (lowest un-occupied molecular orbital) of the electron transport material desirably aligns with the work function of the cathode. Chemical compatibility and sublimation temperature of the materials are also important considerations in selecting the electron and hole transport materials.

It is understood that the efficiency of devices made with the anthracene compounds described herein, can be further improved by optimizing the other layers in the device. For example, more efficient cathodes such as Ca, Ba or LiF can be used. Shaped substrates and novel hole transport materials that result in a reduction in operating voltage or increase quantum efficiency are also applicable. Additional layers can also be added to tailor the energy levels of the various layers and facilitate electroluminescence.

The compounds of the invention often are fluorescent and photoluminescent and can be useful in applications other than OLEDs, such as oxygen sensitive indicators and as fluorescent indicators in bioassays.

### EXAMPLES

The following examples illustrate certain features and advantages of the present invention. They are intended to be illustrative of the invention, but not limiting. All percentages are by weight, unless otherwise indicated.

### Comparative Example A

This example illustrates the preparation of a non-deuterated compound, Comparative Compound A.

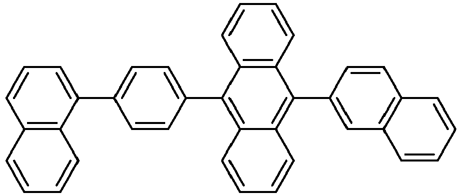

This compound can be prepared according to the following scheme:

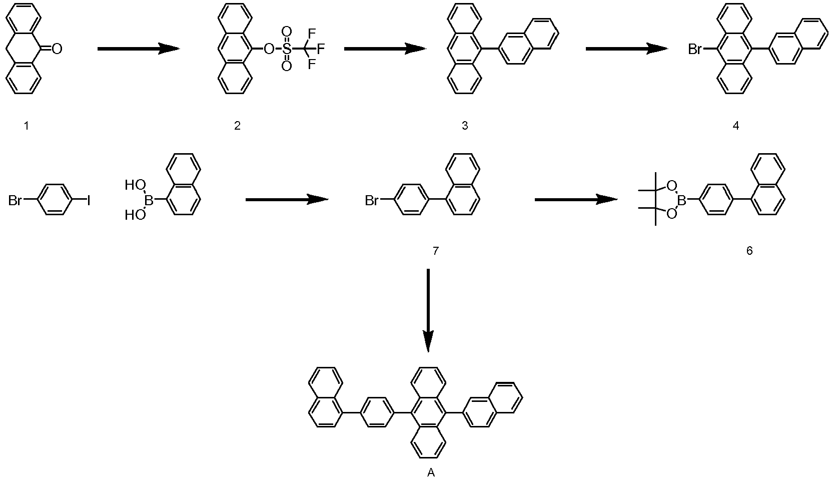

### Synthesis of compound 2:

In a 3L flask fitted with a mechanical stirrer, dropping funnel, thermometer and N₂ bubbler was added anthrone, 54g (275.2mmol) in 1.5L dry methylene chloride. The flask was cooled in an ice bath and 1,8-diazabicyclo[5.4.0]undec-7-ene ("DBU"), 83.7 ml (559.7mmol) was added by dropping funnel over 1.5 hr. The solution turned orange, became opaque, then turned deep red. To the still cooled solution was added triflic anhydride, 58ml (345.0mmol) via syringe over about 1.5hr keeping the temperature of the solution below 5°C. The reaction was allowed to proceed for 3hr at room temperature, after which 1 mL additional triflic anhydride was added and stirring at RT continued for 30min. 500 mL water was added slowly and the layers separated. The aqueous layer was extracted with 3x 200mL dichloromethane ("DCM") and the combined organics dried over magnesium sulfate, filtered and concentrated by rotary evaportaion to give a red oil. Column chromatography on silica gel followed by crystallization from hexanes afforded 43.1 g (43%) of a tan powder

### Synthesis of compound 3:

To a 200 mL Kjeldahl reaction flask equipped with a magnetic stirring bar in a nitrogen-filled glove box were added anthracen-9-yl trifluoromethanesulfonate (6.0 g, 18.40 mmol), Napthalen-2-yl-boronic acid (3.78 g 22.1 mmol), potassium phosphate tribasic (17.50g, 82.0 mmol), palladium(II) acetate (0.41 g, 1.8 mmol), tricyclohexylphosphine (0.52 g, 1.8 mmol) and THF (100 mL). After removal from the dry box, the reaction mixture was purged with nitrogen and degassed water (50 mL) was added by syringe. A condenser was then added and the reaction was refluxed overnight. The reaction was monitored by TLC. Upon completion the reaction mixture was cooled to room temperature. The organic layer was separated and the aqueous layer was extracted with DCM. The organic fractions were combined, washed with brine and dried with magnesium sulfate. The solvent was removed under reduced pressure. The resulting solid was washed with acetone and hexane and filtered. Purification by column chromatography on silica gel afforded 4.03 g (72%) of product as pale yellow crystalline material.

### Synthesis of compound 4:

9-(naphthalen-2-yl)anthracene, 11.17g (36.7mmol) was suspended in 100 mL DCM. N-bromosuccinimide 6.86g (38.5mmol) was added and the mixture stirred with illumination from a 100W lamp. A yellow clear solution formed and then precipitation occurred. The reaction was monitored by TLC. After 1.5 h, the reaction mixture was partially concentrated to remove methylene chloride, and then crystallized from acetonitrile to afford 12.2 g of pale yellow crystals (87%).

### Synthesis of compound 7:

To a 500 mL round bottom flask equipped with a stir bar in a nitrogen-filled glove box were added naphthalen-1-yl-1-boronic (14.2g, 82.6mmol), acid, 1-bromo-2-iodobenzene (25.8g, 91.2 mmol), tetrakis(triphenylphospine) palladium(0) (1.2g, 1.4 mmol), sodium carbonate (25.4g, 240 mmol), and toluene (120 mL). After removal from the dry box, the reaction mixture was purged with nitrogen and degassed water (120 mL) was added by syringe. The reaction flask was then fitted with a condenser and the reaction was refluxed for 15 hours. The reaction was monitored by TLC. The reaction mixture was cooled to room temperature. The organic layer was separated and the aqueous layer was extracted with DCM. The organic fractions were combined and the solvent was removed under reduced pressure to give a yellow oil. Purification by column chromatography using silica gel afforded 13.6 g of a clear oil (58%).

### Synthesis of compound 6:

To a 1-liter flask equipped with a magnetic stirring bar, a reflux condenser that was connected to a nitrogen line and an oil bath, were added 4-bromophenyl-1-naphthalene (28.4g, 10.0 mmol), bis(pinacolate) diboron (40.8g, 16.0 mmol), Pd(dppf)₂Cl₂(1.64 g, 2.0 mmol), potassium acetate (19.7g, 200 mmol), and DMSO (350 mL). The mixture was bubbled with nitrogen for 15 min and then Pd(dppf)₂Cl₂ (1.64 g, 0.002 mol) was added. During the process the mixture turned to a dark brown color gradually. The reaction was stirred at 120°C (oil bath) under nitrogen for 18h. After cooling the mixture was poured into ice water and extracted with chloroform (3x). The organic layer was washed with water (3x) and saturated brine (1 x) and dried with MgSO4. After filtration and removal of solvent, the residue was purified by chromatography on a silica gel column. The product containing fractions were combined and the solvent was removed by rotary evaporation. The resulting white solid was crystallized from hexane/chloroform and dried in a vacuum oven at 40 °C to give the product as white crystalline flakes (15.0 g in 45% yield). 1 H and 13C-NMR spectra are in consistent with the expected structure.

### Synthesis of Comparative Compound A

To a 250 mL flask in glove box were added (2.00g, 5.23 mmol), 4,4,5,5-tetramethyl-2-(4-(naphthalen-4-yl)phenyl)-1,3,2-dioxaborolane (1.90g, 5.74 mmol), tris(dibenzylideneacetone) dipalladium(0) (0.24 g, 0.26 mmol), and toluene (50 mL). The reaction flask was removed from the dry box and fitted with a condenser and nitrogen inlet. Degassed aqueous sodium carbonate (2 M, 20 mL) was added via syringe. The reaction was stirred and heated to 90°C overnight. The reaction was monitored by HPLC. After cooling to room temperature, the organic layer was separated. The aqueous layer was washed twice with DCM and the combined organic layers were concentrated by rotary evaporation to afford a grey powder. Purification by filtration over neutral alumina, hexanes precipitation, and column chromatography over silica gel afforded 2.28 g of a white powder (86%).

The product was further purified as described in published U.S. patent application 2008-0138655, to achieve an HPLC purity of at least 99.9% and an impurity absorbance no greater than 0.01.

### Example 1

This example illustrates the preparation of a compound having Formula I, Compound H1.

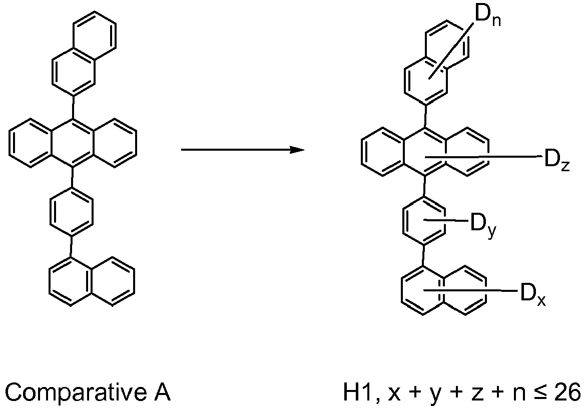

Under an atmosphere of nitrogen, AlCl₃ (0.48g, 3.6 mmol) was added to a perdeuterobenzene or benzene-D6 (C₆D₆) (100 mL) solution of comparative compound A from Comparative Example A (5g, 9.87 mmol). The resulting mixture was stirred at room temperature for six hours after which D₂O (50 mL) was added. The layers were separated followed by washing the water layer with CH₂Cl₂ (2x30 mL). The combined organic layers were dried over magnesium sulfate and the volatiles were removed by rotary evaporation. The crude product was purified via column chromatography. The deuterated product H1 (x+y+n+m = 21-23) was obtained (4.5 g) as a white powder.

The product was further purified as described in published U.S. patent application 2008-0138655, to achieve an HPLC purity of at least 99.9% and an impurity absorbance no greater than 0.01. The material was determined to have the same level of purity as comparative compound A, from above.

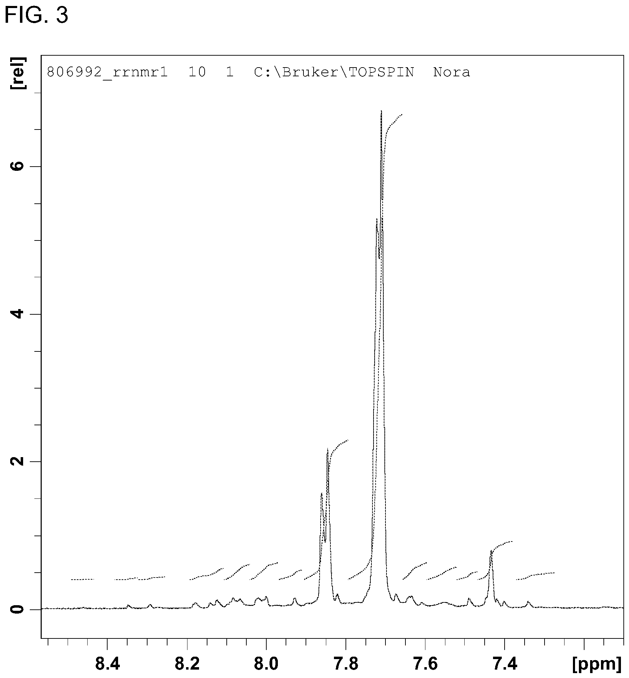

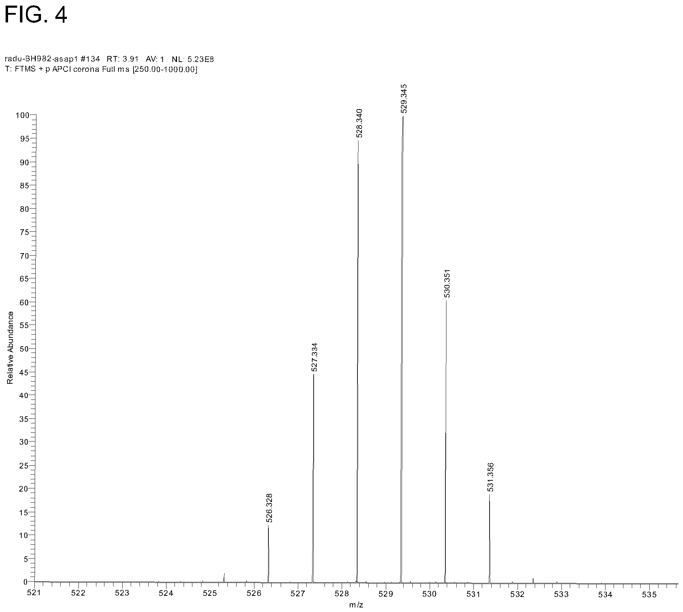

The ¹H NMR (CD₂Cl₂) and ASAP-MS are shown in FIGs 3 and 4, respectively. The compound had the structure given below: 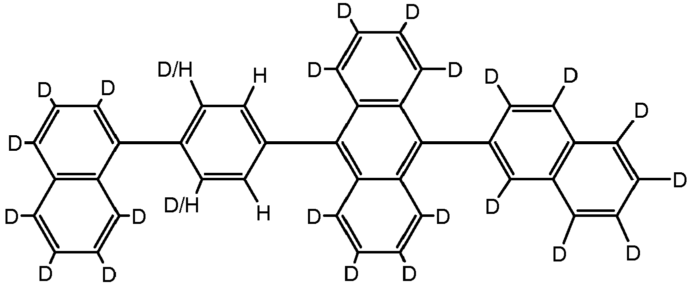 where "D/H" indicates approximately equal probability of H or D at this atomic position. The structure was confimred by ¹H NMR, ¹³C NMR, ²D NMR and ¹H-¹³C HSQC (Heteronuclear Single Quantum Coherence).

### Examples 2 and 3 and Comparative Examples B and C

These examples demonstrate the fabrication and performance of a device with a blue emitter. The following materials were used:
Emitter E1 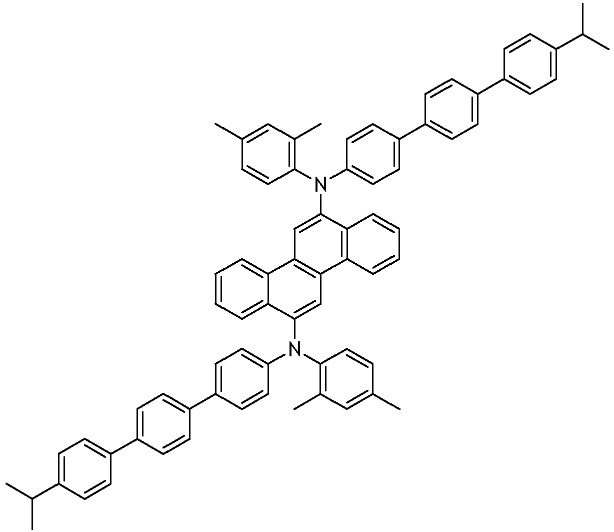
Emitter E2:

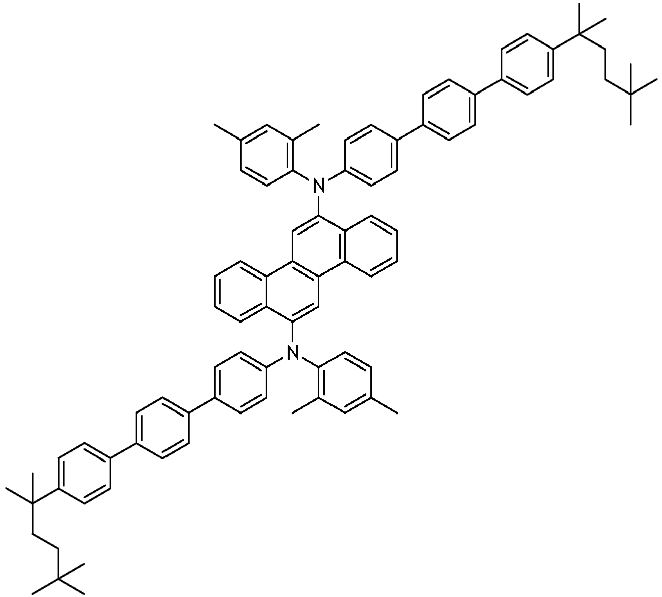

The device had the following structure on a glass substrate:
anode = Indium Tin Oxide (ITO): 50 nm
buffer layer = Buffer 1 (50 nm), which is an aqueous dispersion of an electrically conductive polymer and a polymeric fluorinated sulfonic acid. Such materials have been described in, for example, published U.S. patent applications US 2004/0102577, US 2004/0127637, and US 2005/0205860.
hole transport layer = polymer P1, which is a non-crosslinked arylamine polymer (20 nm)
photoactive layer = 13:1 host:dopant (40 nm), as shown in Table 1
electron transport layer = a metal quinolate derivative (10 nm)
cathode = CsF/Al (1.0/100 nm)

**Table 1. Device Photoactive Layers**

| Example | Host | Dopant |
|---|---|---|
| Comparative B-1 | Comp. Compound A | E1 |
| Comparative B-2 | Comp. Compound A | E1 |
| Comparative B-3 | Comp. Compound A | E1 |
| Comparative B-4 | Comp. Compound A | E1 |
| Ex. 2-1 | H1 | E1 |
| Ex. 2-2 | H1 | E1 |
| Ex. 2-3 | H1 | E1 |
| Ex. 2-4 | H1 | E1 |
| Comparative C-1 | Comp. Compound A | E2 |
| Comparative C-2 | Comp. Compound A | E2 |
| Ex. 3-1 | H1 | E2 |
| Ex. 3-2 | H1 | E2 |

OLED devices were fabricated by a combination of solution processing and thermal evaporation techniques. Patterned indium tin oxide (ITO) coated glass substrates from Thin Film Devices, Inc were used. These ITO substrates are based on Corning 1737 glass coated with ITO having a sheet resistance of 30 ohms/square and 80% light transmission. The patterned ITO substrates were cleaned ultrasonically in aqueous detergent solution and rinsed with distilled water. The patterned ITO was subsequently cleaned ultrasonically in acetone, rinsed with isopropanol, and dried in a stream of nitrogen.

Immediately before device fabrication the cleaned, patterned ITO substrates were treated with UV ozone for 10 minutes. Immediately after cooling, an aqueous dispersion of Buffer 1 was spin-coated over the ITO surface and heated to remove solvent. After cooling, the substrates were then spin-coated with a solution of a hole transport material, and then heated to remove solvent. After cooling the substrates were spin-coated with the emissive layer solution, and heated to remove solvent. The substrates were masked and placed in a vacuum chamber. The electron transport layer was deposited by thermal evaporation, followed by a layer of CsF. Masks were then changed in vacuo and a layer of Al was deposited by thermal evaporation. The chamber was vented, and the devices were encapsulated using a glass lid, dessicant, and UV curable epoxy.

The OLED samples were characterized by measuring their
(1) current-voltage (I-V) curves, (2) electroluminescence radiance versus voltage, and (3) electroluminescence spectra versus voltage. All three measurements were performed at the same time and controlled by a computer. The current efficiency of the device at a certain voltage is determined by dividing the electroluminescence radiance of the LED by the current needed to run the device. The unit is a cd/A. The power efficiency is the current efficiency multiplied by pi, divided by the operating voltage. The unit is Im/W. The device data is given in Table 2.

**TABLE 2. Device Performance**

| Ex. | CIE (x,y) | Voltage (V) | C.E. (cd/A) | E.Q.E. (%) | P.E. (Im/W) | Lifetest current density (mA/cm2) | Lifetest Luminance (nits) | Raw T50 (h) | Projected Lifetime T50 @1000nits |
|---|---|---|---|---|---|---|---|---|---|
| Comp. 3-1 | 0.135, 0.124 | 4.4 | 6.1 | 6.0 | 4.3 | 129 | 6837 | 410 | 10766 |
| Comp. B-2 | 0.135, 0.127 | 4.3 | 6.4 | 6.1 | 4.7 | 125 | 7002 | 410 | 11211 |
| Comp. B-3 | 0.136, 0.123 | 4.4 | 5.7 | 5.6 | 4.1 | 127 | 5906 | 430 | 8804 |
| Comp. B-4 | 0.134, 0.129 | 4.3 | 6.4 | 6.1 | 4.7 | 123 | 7021 | 430 | 11813 |
| Ex. 2-1 | 0.136, 0.119 | 4.4 | 5.7 | 5.7 | 4.1 | 136 | 7040 | **870** | **24010** |
| Ex. 2-2 | 0.137, 0.117 | 4.2 | 5.4 | 5.5 | 4.1 | 126 | 6244 | **830** | **18679** |
| Ex. 2-3 | 0.136, 0.118 | 4.2 | 5.7 | 5.7 | 4.2 | 129 | 6467 | **830** | **19828** |
| Ex. 2-4 | 0.136, 0.118 | 4.3 | 5.5 | 5.6 | 4.1 | 121 | 6148 | **870** | **19071** |
| Comp. C-1 | 0.135, 0.128 | 4.3 | 6.2 | 6.0 | 4.6 | 141 | 8018 | 490 | 16871 |
| Comp. C-2 | 0.135, 0.128 | 4.3 | 6.3 | 6.0 | 4.6 | 125 | 7136 | 510 | 14403 |
| Ex. 3-1 | 0.135, 0.122 | 4.3 | 6.0 | 5.9 | 4.4 | 121 | 6741 | **950** | **24356** |
| Ex. 3-2 | 0.135, 0.124 | 4.2 | 6 | 5.8 | 4.4 | 126 | 6974 | **930** | **25257** |

| | | | | | | | | | |
|---|---|---|---|---|---|---|---|---|---|
| * All data @ 1000 nits, CE = current efficiency; CIEx and CIEy are the x and y color coordinates according to the C.I.E. chromaticity scale (Commission Internationale de L'Éclairage, 1931). RawT50 is the time in hours for a device to reach one-half the initial luminance at the life test luminance given. Projected T50 is the projected lifetime at 1000 nits using an accelerator factor of 1.7. | | | | | | | | | |

It can be seen that with the deuterated host of the invention, the lifetime of devices is greatly increased. When emitter E1 was used, the comparative devices with a non-deuterated host (Comparative examples B-1 through B-4) had an average raw T50 of 420 hours. With the deuterated analog host H1 (Examples 2-1 through 2-4), the devices have an average raw T50 of 850 hours. When emitter E2 was used, the comparative devices (C-1 and C-2) had an average raw T50 of 500 hours. With the deuterated analog host H1 (3-1 and 3-2), the average raw T50 was 940 hours.

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification and figures are to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

## Claims

1. An aryl-substituted anthracene compound having Formula I: 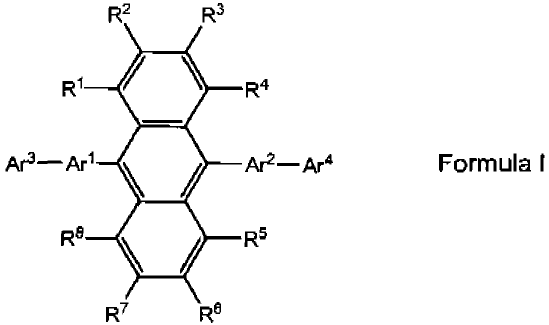 wherein:
R¹ through R⁸ are the same or different at each occurrence and are selected from the group consisting of H, D, alkyl, alkoxy, aryl, aryloxy, diarylamino, siloxane, and silyl;
Ar¹ and Ar² are the same or different and are selected from the group consisting of aryl groups; and
Ar³ and Ar⁴ are the same or different and are selected from the group consisting of H, D, and aryl groups; **characterised in that** at least one of R¹ through R⁸ is D.

2. The compound of Claim 1, wherein R¹ through R⁸ are selected from H and D.

3. The compound of Claim 1, wherein at least one of R¹ through R⁸ is selected from alkyl, alkoxy, aryl, aryloxy, diarylamino, siloxane, and silyl, and the remainder of R¹ through R⁵ are selected from H and D.

4. The compound of Claim 1, wherein at least one of Ar¹ through Ar⁴ is a deuterated aryl.

5. The compound of Claim 1, wherein Ar³ and Ar⁴ are selected from D and deuterated aryls.

6. The compound of Claim 1, wherein Ar¹ and Ar² are selected from the group consisting of phenyl, naphthyl, phenanthryl, and anthracenyl.

7. The compound of Claim 1, wherein Ar³ and Ar⁴ are selected from the group consisting of phenyl, naphthyl, phenanthryl, anthracenyl, phenylnaphthylene, naphthylphenylene, and a group having Formula II: 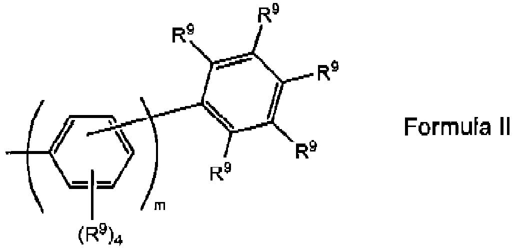 where:
R⁹ is the same or different at each occurrence and is selected from the group consisting of H, D, alkyl, alkoxy, diarylamino, siloxane and silyl, or adjacent R⁹ groups may be joined together to form an aromatic ring; and
m is the same or different at each occurrence and is an integer from 1 to 6.

8. The compound of Claim 1, wherein Ar³ and Ar⁴ are selected from the group consisting of phenyl, naphthyl, phenylnaphthylene, naphthylphenylene, and a group having Formula III: 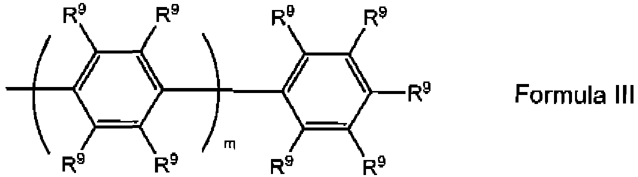 where:
R⁹ is the same or different at each occurrence and is selected from the group consisting of H, D, alkyl, alkoxy, siloxane and silyl, or adjacent R⁹ groups may be joined together to form an aromatic ring; and
m is the same or different at each occurrence and is an integer from 1 to 6.

9. An organic electronic device comprising a first electrical contact layer, a second electrical contact layer, and at least one active layer therebetween, wherein the active layer comprises an aryl-substituted anthracene compound according to any preceding claim.

10. The device of claim 9, wherein the aryl-substituted anthracene compound hosts a photoactive material.

11. The device of Claim 9, wherein the active layer consists essentially of the compound having Formula I and one or more electroluminescent compounds.

12. The device of Claim 9, wherein the electroluminescent compound is selected from the group consisting of amino-substituted chrysenes and amino-substituted anthracenes.

13. The device of Claim 9, further comprising a buffer layer between the first electrical contact layer and the active layer, preferably wherein the buffer layer comprises at least one electrically conductive polymer and at least one fluorinated acid polymer.

## Patentansprüche

1. Arylsubstituierte Anthracenverbindung, die die Formel I aufweist: 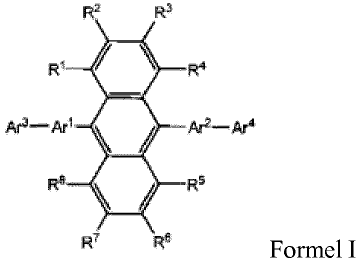 wobei
R₁ bis R₈ bei jedem Vorkommen gleich oder verschieden und aus der Gruppe ausgewählt sind bestehend aus H, D, Alkyl, Alkoxy, Aryl, Aryloxy, Diarylamino, Siloxan und Silyl;
Ar¹ und Ar² gleich oder verschieden und aus der Gruppe ausgewählt sind bestehend aus Arylgruppen; und
Ar³ und Ar⁴ gleich oder verschieden und aus der Gruppe ausgewählt sind bestehend aus H, D und Arylgruppen;
**dadurch gekennzeichnet, dass** mindestens eines von R¹ bis R⁸ D ist.

2. Verbindung nach Anspruch 1, wobei R¹ bis R⁸ aus H und D ausgewählt sind.

3. Verbindung nach Anspruch 1, wobei mindestens eines von R¹ bis R⁸ unter Alkyl, Alkoxy, Aryl, Aryloxy, Diarylamino, Siloxan und Silyl ausgewählt ist und der Rest von R¹ bis R⁸ aus H und D ausgewählt ist.

4. Verbindung nach Anspruch 1, wobei mindestens eines von Ar¹ bis Ar⁴ ein deuteriertes Aryl ist.

5. Verbindung nach Anspruch 1, wobei Ar³ und Ar⁴ aus D und deuterierten Arylen ausgewählt sind.

6. Verbindung nach Anspruch 1, wobei Ar¹ und Ar² aus der Gruppe ausgewählt sind bestehend aus Phenyl, Naphthyl, Phenanthryl und Anthracenyl.

7. Verbindung nach Anspruch 1, wobei Ar³ und Ar⁴ aus der Gruppe ausgewählt sind bestehend aus Phenyl, Naphthyl, Phenanthryl, Anthracenyl, Phenylnaphthylen, Naphthylphenylen und einer Gruppe, die die Formel II aufweist: 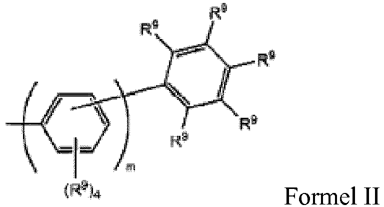 wobei:
R⁹ bei jedem Vorkommen gleich oder verschieden und aus der Gruppe ausgewählt ist bestehend aus H, D, Alkyl, Alkoxy, Diarylamino, Siloxan und Silyl oder anliegende R⁹-Gruppen miteinender verbunden sein können, um einen aromatischen Ring zu bilden; und
m bei jedem Vorkommen gleich oder verschieden und eine ganze Zahl von 1 bis 6 ist.

8. Verbindung nach Anspruch 1, wobei Ar³ und Ar⁴ aus der Gruppe ausgewählt sind bestehend aus Phenyl, Naphthyl, Phenylnaphthylen, Naphthylphenylen und einer Gruppe, die die Formel III aufweist: 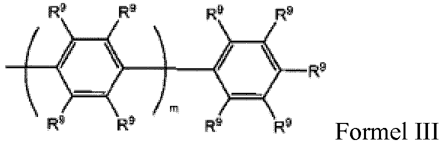 wobei:
R⁹ bei jedem Vorkommen gleich oder verschieden und aus der Gruppe ausgewählt ist bestehend aus H, D, Alkyl, Alkoxy, Siloxan und Silyl oder anliegende R⁹-Gruppen miteiander verbunden sein können, um einen aromatischen Ring zu bilden; und
m bei jedem Vorkommen gleich oder verschieden und eine ganze Zahl von 1 bis 6 ist.

9. Organische elektronische Vorrichtung umfassend eine erste elektrische Kontaktschicht, eine zweite elektrische Kontaktschicht und mindestens eine aktive Schicht dazwischen, wobei die aktive Schicht eine arylsubstituierte Anthracenverbindung nach einem der vorhergehenden Ansprüche umfasst.

10. Vorrichtung nach Anspruch 9, wobei die arylsubstituierte Anthracenverbindung ein fotoaktives Material unterbringt.

11. Vorrichtung nach Anspruch 9, wobei die aktive Schicht im Wesentlichen aus der Verbindung, die die Formel I aufweist, und einer oder mehreren elektrolumineszierenden Verbindungen besteht.

12. Vorrichtung nach Anspruch 9, wobei die elektrolumineszierende Verbindung aus der Gruppe ausgewählt ist bestehend aus amino substituierten Chrysenen und aminosubstituierten Anthracenen.

13. Vorrichtung nach Anspruch 9, ferner eine Pufferschicht zwischen der ersten elektrischen Kontaktschicht und der aktiven Schicht umfassend, wobei bevorzugt die Pufferschicht mindestens ein elektrisch leitfähiges Polymer und mindestens ein fluoriertes saures Polymer umfasst.

## Revendications

1. Composé anthracène substitué par un groupe aryle de Formule I: 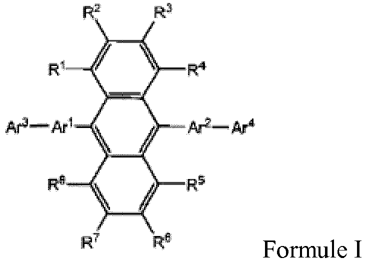 dans laquelle:
R¹ jusqu'à R⁸ sont identiques ou différents à chaque apparition et sont sélectionnés dans le groupe constitué de l'atome H, D, du groupe alkyle, alcoxy, aryle, aryloxy, diarylamino, siloxane, et silyle;
Ar¹ et Ar² sont identiques ou différents et sont sélectionnés dans le groupe constitué des groupes aryle; et
Ar³ et Ar⁴ sont identiques ou différents et sont sélectionnés dans le groupe constitué de l'atome H, D, et des groupes aryle;
**caractérisé en ce qu'**au moins l'un de R¹ jusqu'à R⁸ est D.

2. Composé selon la revendication 1, dans lequel les R¹ jusqu'à R⁸ sont sélectionnés parmi l'atome H et D.

3. Composé selon la revendication 1, dans lequel au moins l'un de R¹ jusqu'à R⁸ est sélectionné parmi un groupe alkyle, alcoxy, aryle, aryloxy, diarylamino, siloxane, et silyle, et le reste de R¹ jusqu'à R⁸ est sélectionné parmi l'atome H et D.

4. Composé selon la revendication 1, dans lequel au moins l'un de Ar¹ jusqu'à Ar⁴ est un groupe aryle deutérié.

5. Composé selon la revendication 1, dans lequel les Ar³ et Ar⁴ sont sélectionnés parmi D et les groupes aryle deutériés.

6. Composé selon la revendication 1, dans lequel les Ar¹ et Ar² sont sélectionnés dans le groupe constitué du groupe phényle, naphtyle, phénanthryle, et anthracényle.

7. Composé selon la revendication 1, dans lequel les Ar³ et Ar⁴ sont sélectionnés dans le groupe constitué du groupe phényle, naphtyle, phénanthryle, anthracényle, phénylnaphtylène, naphtylphénylène, et un groupe de Formule II: 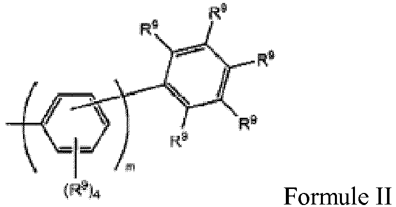 où:
R⁹ est identique ou différent à chaque apparition et est sélectionné dans le groupe constitué de l'atome H, D, des groupes alkyle, alcoxy, diarylamino, siloxane et silyle, ou les groupes R⁹ adjacents peuvent être joints ensemble pour former un cycle aromatique; et
m est identique ou différent à chaque apparition et est un nombre entier d'une valeur de 1 à 6.

8. Composé selon la revendication 1, dans lequel les Ar³ et Ar⁴ sont sélectionnés dans le groupe constitué du groupe phényle, naphtyle, phénylnaphtylène, naphtylphénylène, et d'un groupe de Formule III: 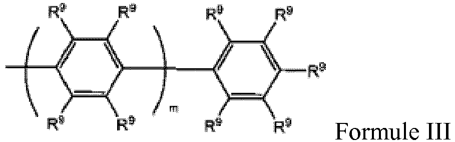 où:
R⁹ est identique ou différent à chaque apparition et est sélectionné dans le groupe constitué de l'atome H, D, des groupes alkyle, alcoxy, siloxane et silyle, ou les groupes R⁹ adjacents peuvent être joints ensemble pour former un cycle aromatique; et
m est identique ou différent à chaque apparition et est un nombre entier d'une valeur de 1 à 6.

9. Dispositif électronique organique comprenant une première couche de contact électrique, une seconde couche de contact électrique, et au moins une couche active entre elles, la couche active comprenant un composé anthracène substitué par un groupe aryle selon l'une quelconque des revendications précédentes.

10. Dispositif selon la revendication 9, dans lequel le composé anthracène substitué par un groupe aryle héberge un matériau photoactif.

11. Dispositif selon la revendication 9, la couche active étant constituée essentiellement du composé de Formule 1 et d'un ou plusieurs composés électroluminescents.

12. Dispositif selon la revendication 9, dans lequel le composé électroluminescent est sélectionné dans le groupe constitué des chrysènes substitués par un groupe amino et des anthracènes substitués par un groupe amino.

13. Dispositif selon la revendication 9, comprenant en outre une couche tampon entre la première couche de contact électrique et la couche active, préférablement dans lequel la couche tampon comprend au moins un polymère électriquement conducteur et au moins un polymère d'acide fluoré.
